(19) Europäisches Patentamt
European Patent Office
Office européen des brevets

(11) Publication number: **0 157 538 A2**

(12) **EUROPEAN PATENT APPLICATION**

(21) Application number: **85301912.3**

(22) Date of filing: **19.03.85**

(51) Int. Cl.⁴: **C 07 D 501/46**

(30) Priority: **26.03.84 US 593441**

(43) Date of publication of application: **09.10.85 Bulletin 85/41**

(84) Designated Contracting States: **AT BE CH DE FR GB IT LI LU NL SE**

(71) Applicant: **ELI LILLY AND COMPANY, Lilly Corporate Center, Indianapolis Indiana 46285 (US)**

(72) Inventor: **Chou, Ta-Sen, 745 Canyon Road, Indianapolis Indiana 46217 (US)**

(74) Representative: **Hudson, Christopher Mark et al, Erl Wood Manor, Windlesham Surrey GU20 6PH (GB)**

(54) **Improvements in or relating to crystalline ceftazidime salts.**

(57) Ceftazidime bishydrobromide crystalline monohydrates, useful for preparing crystalline ceftazidime pentahydrate, are provided.

EP 0 157 538 A2

# IMPROVEMENTS IN OR RELATING TO CRYSTALLINE CEFTAZIDIME SALTS

In the preparation of important antibiotics, researchers require stable, highly purified crystalline forms to ensure the highest purity possible.

The development of commercially-useful crystalline forms requires extensive experimentation and time expenditures because, as one skilled in the art will know, until a particular crystalline form is actually observed, one can not predict its stability or crystalline form, much less that a crystalline compound would result from the particular steps involved in its production.

This invention relates to the crystalline salt forms of the cephalosporin antibiotic, ceftazidime. In particular, the dihydrobromide salt and crystalline dihydrobromide monohydrates of ceftazidime are provided.

The antibiotic ceftazidime is disclosed in U.S. Patent No. 4,258,041, March 24, 1981, and has the chemical name (6R,7R)-7-[(Z)-2-(2-aminothiazol-4-yl)-2-(2-carboxyprop-2-oxyimino)acetamido]-3-(1-pyridinium-methyl)-3-cephem-4-carboxylate. Ceftazidime is a potent broad spectrum semi-synthetic antibiotic which can be formulated as the crystalline pentahydrate. Ceftazidime pentahydrate has been prepared with ceftazidime bishydrochloride salt as disclosed in U.S. Patent No. 4,329,453, May 11, 1982.

A crystalline ceftazidime bishydrochloride salt form is disclosed in U.K. Application No. 2064513.

This invention provides ceftazidime bishydrobromide salt and crystalline monohydrate forms of the ceftazidime bishydrobromide salt. Crystalline forms of the bishydrobromide salt are obtained directly from the formic acid-HBr deblocking of the tritylamino and t-butyl ester protected ceftazidime reaction mixture. The crystalline monohydrates are stable and are useful in the preparation of the known crystalline ceftazidime pentahydrate.

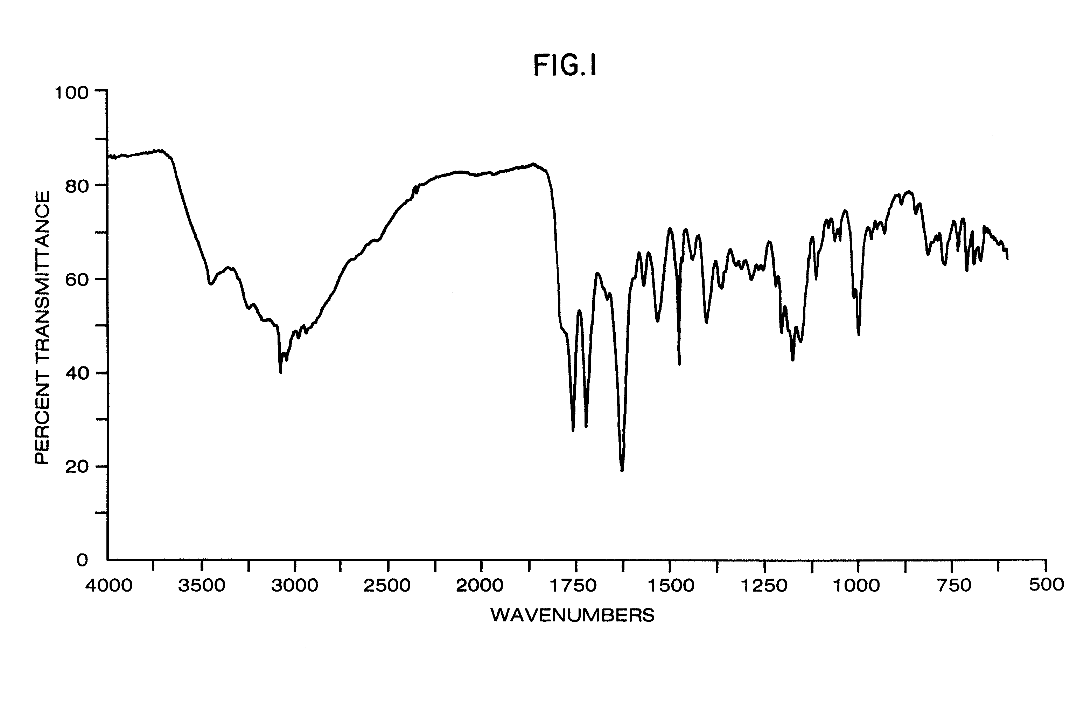

Figure 1 is the infrared absorption spectrum of ceftazidime bishydrobromide α-monohydrate (KBr pellet).

This invention provides crystalline monohydrate forms of ceftazidime bishydrobromide. In particular, the form designated as the α-monohydrate is a preferred form. The crystalline monohydrates are characterized by their X-ray diffraction patterns and infrared (IR) spectra.

In addition, there is provided a process for preparing crystalline ceftazidime bishydrobromide monohydrate which comprises diluting, to induce crystallization, a solution of ceftazidime bishydrobromide with a water miscible organic solvent.

The following diffraction pattern of the α-monohydrate form was obtained using a copper target X-ray tube with a nickel filter and a 114.6 mm DeBye-Scherrer camera. The "d" refers to interplanar spacings and "$I/I_1$" to the relative intensities.

α-Monohydrate

| d | $I/I_1$ |
|---|---|
| 14.85 | .17 |
| 10.65 | .50 |
| 9.94 | .08 |
| 8.08 | .29 |
| 7.66 | .29 |
| 6.71 | .25b |
| 5.74 | .38 |
| 5.37 | .04 |
| 5.16 | .04 |
| 4.77 | .25 |
| 4.37 | .83 |
| 4.22 | 1.00 |
| 4.04 | .04 |
| 3.89 | 1.00 |
| 3.78 | .17 |
| 3.56 | .17 |
| 3.35 | .21 |
| 3.29 | .25 |
| 3.11 | .25 |
| 2.93 | .29 |
| 2.88 | .25 |
| 2.79 | .08 |
| 2.74 | .17 |
| 2.70 | .04 |
| 2.58 | .08 |
| 2.54 | .13 |
| 2.45 | .13 |
| 2.24 | .17 |
| 2.14 | .08 |
| 2.09 | .08 |

The preferred α monohydrate form analyzes correctly for the percent elemental composition for ceftazidime bishydrobromide monohydrate, having the empirical formula of $C_{22}H_{22}N_6O_7S_2 \cdot 2HBr \cdot H_2O$. The significant IR absorption maxima of the monohydrate, as shown in the drawing, are listed below in Table I. The spectra were obtained on a Nicolet model 10 MX spectrophotometer.

Table I

Ceftazidime bishydrobromide monohydrate
Infrared Absorption Spectrum
(KBr pellet) $cm^{-1}$

| α-monohydrate |
|---|
| 1176.7 |
| 1480.5 |
| 1630.0 |
| 1725.5 |
| 1760.2 |
| 2982.2 |
| 3034.2 |
| 3045.8 |
| 3056.4 |
| 3077.6 |

The preferred α-monohydrate crystalline form of this invention can be obtained by deblocking the ceftazidine intermediate, (6R,7R)-7-[(Z)-2-(2-tritylamino-1,3-thiazol-4-yl)-2-t-butyloxycarbonylprop-2-oxyimino]acetamido]-3-(1-pyridiniummethyl)-3-cephem-4-carboxylate. This intermediate is reacted with formic acid (98%) and 48% hydrobromic acid to remove both the t-butyl carboxy-protecting group and the trityl amino-protecting group. The deblocked ceftazidime intermediate forms, in solution, the bishydrobromide salt. The following reaction scheme illustrates the deblocking reaction:

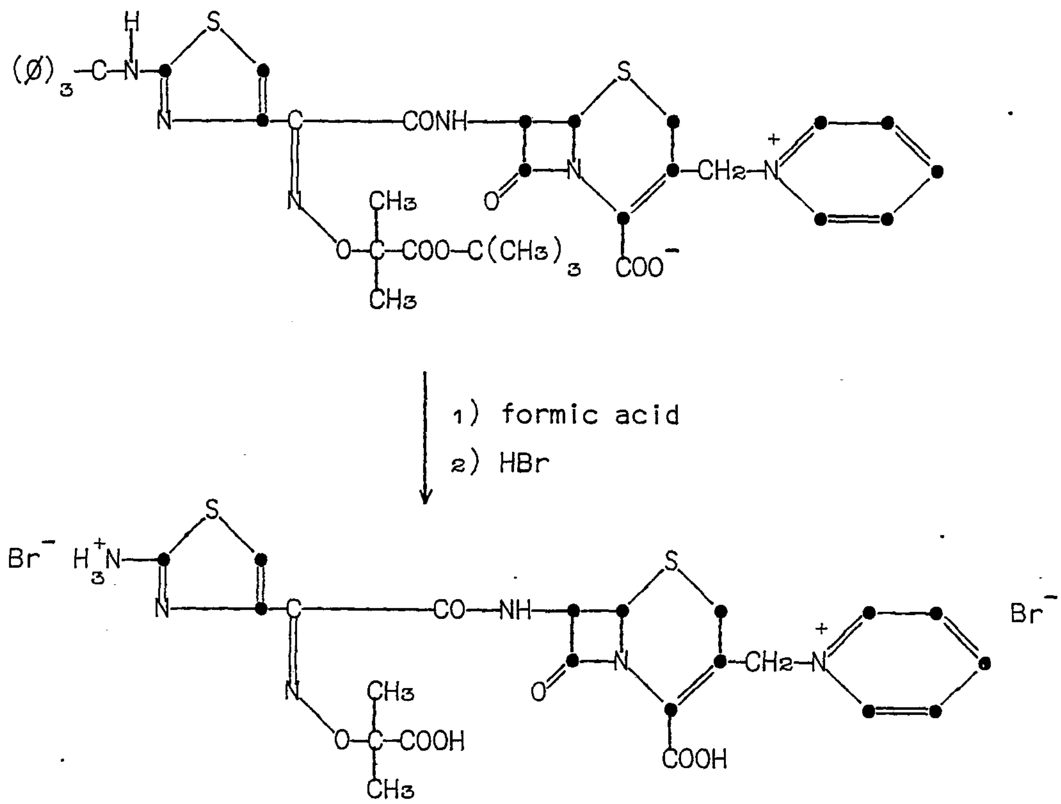


The crystalline monohydrates of the salt are isolated from the reaction mixture by filtering the deblocking reaction mixture to remove the insoluble triphenylcarbinol and the filtrate then is diluted with a water miscible organic solvent to induce crystallization. The α-monohydrate is obtained with dilution by acetone, methylethyl ketone, cyclohexanone, and

isopropanol. The dilution of the filtered reaction mixture generally is performed at room temperature and, following dilution, the crystallization mixture may be cooled to a temperature between about 10°C to 20°C to assist crystallization. In general, the filtered reaction mixture is diluted to near the cloud point or to the cloud point and the solution is chilled with stirring or allowed to stir at room temperature until crystallization occurs. With some reaction mixtures the salt may precipitate as a gum or heavy oil. The gum or oil may be induced to crystallize by redissolving the gum or oil by addition to the mixture of formic acid followed by additional diluting solvent. Alternatively, the gum or oil is separated from the mother liquor, redissolved in aqueous formic acid, and the solution diluted with the appropriate water miscible organic solvent. The crystalline ceftazidime bishydrobromide monohydrate is separated from the mother liquor by filtration and is washed with the same water miscible organic solvent used to induce crystallization. The crystalline salt then is dried at room temperature under vacuum.

The α-monohydrate form of the bishydrobromide salt may be isolated from the unfiltered deblocking reaction mixture. In this instance the deblocking reaction mixture containing the insoluble triphenylcarbinol is diluted with methylethyl ketone to first solubilize the carbinol. Further dilution with methylethyl ketone or acetone then induces crystallization of the dihydrobromide salt.

The crystalline salt monohydrates of the invention also may be prepared with ceftazidime pentahydrate or with amorphous hydrated ceftazidime. In this preparation the pentahydrate or amorphous hydrate is converted to the bishydrobromide salt with excess 48% hydrobromic acid and the acid solution is diluted with the appropriate water miscible organic solvent to induce crystallization as described earlier.

Preferred water miscible organic solvents for use in inducing crystallization of the α-monohydrate are methylethyl ketone and cyclohexanone. These solvents afford the largest crystals having well-defined structures.

The crystalline hydrates of this invention remain stable under ordinary storage conditions for several months.

The crystalline bishydrobromide monohydrates are useful in the preparation of crystalline ceftazidime pentahydrate. The bishydrobromide monohydrate is dissolved in water or an aqueous solvent system containing a water miscible solvent. The pH of the solution is adjusted with base to about 3.5 to about 4.0 and the pentahydrate form of ceftazidime crystallizes from the solution.

The crystalline salts of this invention also may be used as active forms of ceftazidime for administration by the parenteral routes, e.g., intramuscular injection. They can be formulated with pharmaceutically-acceptable carriers or diluents, e.g., water-for-injection, 0.9% saline, or 5% glucose for injection. Thus, there is provided a pharmaceutical formulation

which comprises as an active ingredient, ceftazidime bishydrobromide monohydrate, as described earlier, associated with one or more pharmaceutically-acceptable carriers or diluents therefor.

The following non-limiting examples are provided to further describe the invention.

Example 1

Ceftazidime dihydrobromide α-monohydrate

(6R,7R)-7-[(Z)-2-(2-tritylaminothiazol-4-yl)-2-(2-t-butyloxycarbonylprop-2-oxyimino)acetamido]-3-(1-pyridiniummethyl)-3-cephem-4-carboxylate, 18.5 g (0.02M), was added to 40 ml of 98% formic acid (53 eq.). During the addition, the mixture was maintained below a temperature of 25°C by ice cooling. The cooling bath was removed and the mixture was stirred for 2 hours at room temperature. After recooling to about 10°C to about 15°C 11.4 ml of 48% hydrobromic acid were added dropwise to the solution. After acid addition was complete the solution was stirred for 2 hours at room temperature. The reaction mixture was not filtered to remove triphenylcarbinol but, instead, 150 ml of methylethyl ketone (MEK) were added slowly to the mixture, producing a clear solution. An additional 304 ml of MEK were added over 45 minutes. The product which formed was an insoluble oily gum to which an additional 100 ml of MEK were added. After stirring the reaction mixture for 30 minutes at room temperature the mixture was warmed to a temperature of about 70°C to about 75°C. The gum began to crystallize in the warm mixture which

was then cooled to a temperature of 5-10°C. After the mixture was stirred in the cold for 30 minutes the crystals were filtered, washed with 50 ml of cold MEK, and dried *in vacuo* at room temperature to afford 4.48 g of the bishydrobromide α-monohydrate. A second fraction, 6.82 g of the crystalline salt (darker in color) also was obtained.

Elemental analysis of the first fraction after drying the sample at 40°C, calculated for $C_{22}H_{22}N_6O_7S_2 \cdot 2HBr \cdot H_2O$:

Theory: C, 36.38; H, 3.61; N, 11.57; O, 17.62; S, 8.83; Br, 22.00

Found: C, 36.12; H, 3.36; N, 11.33; O, 17.76; S, 8.72; Br, 22.26.

## Example 2

### Ceftazidime bishydrobromide α-monohydrate

The trityl amino-protected and t-butyl ester protected ceftazidime used in the preceeding example, 18.0 g, was added to 40 ml of 98% formic acid. The temperature of the mixture was maintained below about 25°C with an ice bath. The reaction mixture was stirred for 30 minutes and 11.8 ml of 48% hydrobromic acid were added dropwise with stirring to the mixture. The acid mixture was stirred for 3.5 hours and was filtered to remove the insoluble triphenylmethanol. The filtrate was divided into two equal volumes and one-half (31 ml) was added dropwise with stirring to 185 ml of methylethyl ketone. As the addition of the filtrate proceeded crystalline monohydrate began to precipitate. An

additional 50 ml of methylethyl ketone were added and the mixture was stirred at room temperature for 1.5 hours. The crystalline α-monohydrate was filtered, washed with 50 ml of the ketone, and dried at room temperature in vacuo. The white crystalline α-monohydrate weighed 5.50 g.

The other half of the filtered deblocking reaction mixture (31 ml) was added dropwise with stirring to 185 ml of cyclohexanone at room temperature. Some white precipitate formed and when about one-half of the filtrate had been added some gum formation was observed. The rest of the filtrate was added and the mixture was stirred for about 2 hours with some crystal formation. The mixture was stirred overnight with formation of a quantity of dense crystals. The crystals were filtered, washed with 50 ml of cyclohexanone and dried without heating in vacuo. There were obtained 4.68 g of the crystalline α-monohydrate melting at about 177°C with decomposition.

Elemental analysis of a sample of the product after drying at 40°C gave the following results, calculated for $C_{22}H_{22}N_6O_7S_2 \cdot 2HBr \cdot H_2O$:

Theory: C, 36.38; H, 3.61; N, 11.57; O, 17.62; S, 8.83; Br, 22.00

Found: C, 36.67; H, 3.57; N, 11.47; O, 17.52; S, 8.97; Br, 21.89.

Mass Spectral Analysis: M/e 547.

m.p.: starts to decompose at 175°C.

## Example 3

Conversion of Ceftazidime bishydrobromide monohydrate to Ceftazidime pentahydrate

To a beaker containing 36 ml of water chilled to 5-10°C was added 9.0 g of ceftazidime bishydrobromide monohydrate. The mixture was stirred to obtain a solution, after which the pH is adjusted to 2.2 with 2N NaOH. The resulting murky solution then was filtered through a pad of acid washed Celite. The temperature of the filtrate was maintained at about 13°C to 15°C and the pH was adjusted to about 3.75 with 2N NaOH. The solution, maintained at 13-15°C was seeded and stirring was continued. After 2 hours crystallization was complete. The mixture was cooled to 0°-5°C and stirred for 3 hours, filtered and washed with 40 ml each of cold water and acetone. The resulting crystals (5.92 g) were allowed to air dry overnight. The corrected yield of product was 77%.

## CLAIMS

1. Ceftazidime bishydrobromide.

2. Crystalline ceftazidime bishydrobromide.

3. Crystalline ceftazidime bishydrobromide monohydrate.

4. Crystalline ceftazidime bishydrobromide monohydrate having the following X-ray diffraction pattern:

| d | $I/I_1$ | d | $I/I_1$ |
|---|---|---|---|
| 14.85 | .17 | 2.79 | .08 |
| 10.65 | .50 | 2.74 | .17 |
| 9.94 | .08 | 2.70 | .04 |
| 8.08 | .29 | 2.58 | .08 |
| 7.66 | .29 | | |
| | | 2.54 | .13 |
| 6.71 | .25b | 2.45 | .13 |
| 5.74 | .38 | 2.24 | .17 |
| 5.37 | .04 | 2.14 | .08 |
| 5.16 | .04 | 2.09 | .08 |
| 4.77 | .25 | | |
| 4.37 | .83 | | |
| 4.22 | 1.00 | | |
| 4.04 | .04 | | |
| 3.89 | 1.00 | | |
| 3.78 | .17 | | |
| 3.56 | .17 | | |
| 3.35 | .21 | | |
| 3.29 | .25 | | |
| 3.11 | .25 | | |
| 2.93 | .29 | | |
| 2.88 | .25 | | |

5. The α-form of crystalline ceftazidime bishydrobromide monohydrate.

6. A process for preparing crystalline ceftazidime bishydrobromide monohydrate which comprises diluting to induce crystallization, a solution of ceftazidime bishydrobromide with a water miscible organic solvent.

7. A process as claimed in claim 6 in which the ceftazidime bishydrobromide is prepared by treating (6R,7R)-7-[(Z)-2-(2-tritylamino-1,3-thiazol-4-yl)-2-t-butyloxycarbonylprop-2-oxyimino]acetamido-3-(1-pyridinium-methyl)-3-cephem-4-carboxylate with formic acid and hydrobromic acid.

8. A process as claimed in claim 6 or 7 in which the water miscible organic solvent is selected from methyl ethyl ketone, cyclohexanone, acetone or isopropanol.

9. A process as claimed in any one of claims 6 to 8 in which the water miscible solvent is methyl ethyl ketone or cyclohexanone.

10. Ceftazidime bishydrobromide monohydrate, as claimed in any one of claims 2 to 5, for use as an antibiotic in the chemotherapy of a warm-blooded animal.

11. A pharmaceutical formulation which comprises as an active ingredient, ceftazidime bishydrobromide monohydrate, as claimed in any one of claim 2 to 5, associated with one or more pharmaceutically-acceptable carriers or diluents therefor.

12. A process for preparing ceftazidime pentahydrate which comprises preparing ceftazidime bishydrobromide monohydrate salt and converting the salt to ceftazidime pentahydrate.

CLAIMS

1. A process for preparing crystalline ceftazidime bishydrobromide monohydrate which comprises diluting to induce crystallization, a solution of ceftazidime bishydrobromide with a water miscible organic solvent.

2. A process as claimed in claim 1 for preparing crystalline ceftazidime bishydrobromide monohydrate having the following X-ray diffraction pattern:

| d | $I/I_1$ | d | $I/I_1$ |
|---|---|---|---|
| 14.85 | .17 | 2.79 | .08 |
| 10.65 | .50 | 2.74 | .17 |
| 9.94 | .08 | 2.70 | .04 |
| 8.08 | .29 | 2.58 | .08 |
| 7.66 | .29 | | |
| | | 2.54 | .13 |
| 6.71 | .25b | 2.45 | .13 |
| 5.74 | .38 | 2.24 | .17 |
| 5.37 | .04 | 2.14 | .08 |
| 5.16 | .04 | 2.09 | .08 |
| 4.77 | .25 | | |
| 4.37 | .83 | | |
| 4.22 | 1.00 | | |
| 4.04 | .04 | | |
| 3.89 | 1.00 | | |
| 3.78 | .17 | | |
| 3.56 | .17 | | |
| 3.35 | .21 | | |
| 3.29 | .25 | | |
| 3.11 | .25 | | |
| 2.93 | .29 | | |
| 2.88 | .25 | | |

3. A process as claimed in claim 1 or 2 for preparing the α-form of crystalline ceftazidime bishydrobromide monohydrate.

4. A process as claimed in any one of claims 1 to 3 in which the ceftazidime bishydrobromide is prepared by treating (6R,7R)-7-[(Z)-2-(2-tritylamino-1,3-thiazol-4-yl)-2-t-butyloxycarbonylprop-2-oxyimino]-acetamido-3-(1-pyridiniummethyl)-3-cephem-4-carboxylate with formic acid and hydrobromic acid.

5. A process as claimed in claim 4 in which the water miscible organic solvent is selected from methyl ethyl ketone, cyclohexanone, acetone or isopropanol.

6. A process as claimed in any one of claims 1 to 5 in which the water miscible solvent is methyl ethyl ketone or cyclohexanone.

7. A process for preparing ceftazidime pentahydrate which comprises preparing ceftazidime bishydrobromide monohydrate salt and converting the salt to ceftazidime pentahydrate.

8. Ceftazidime bishydrobromide monohydrate whenever prepared according to a process as claimed in any one of claims 1 to 6.

0157538

1/1

FIG.I